# DEMANDE DE BREVET EUROPEEN

(11) **EP 1 205 164 A1**
(43) Date de publication de la demande: **15.05.2002**
(21) Numéro de dépôt: 01420203.0
(22) Date de dépôt: 10.10.2001
(51) Int. Cl.: A61F 2/36

(54) **Prothèse de hanche**

(30) Priorité: 07.11.2000 FR 0014239
(71) Demandeur: Biomet Merck France, 26000 Valence (FR); Champion, Philippe Jean, 41100 Vendome (FR); Gueriot, Sylvain, 59500 Douai (FR); Jouanin, Thierry, 26200 Montelimar (FR); LePage, Jérôme, 92210 Saint Cloud (FR); Martin, Guy, 22360 Langueux (FR); Meignie, Stéphane, 17000 La Rochelle (FR); Simottel, Jean-Claude, 76850 Cottevrard (FR); Vinh, Tho-Son, 75014 Paris (FR)
(72) Inventeur: Champion, Philippe Jean, 41100 Vendome (FR); Gueriot, Sylvain, 59500 Douai (FR); Jouanin, Thierry, 26200 Montelimar (FR); LePage, Jérôme, 92210 Saint Cloud (FR); Martin, Guy, 22360 Langueux (FR); Meignie, Stéphane, 17000 La Rochelle (FR)
(74) Mandataire: Vuillermoz, Bruno

(57) **Abrégé**

Prothèse de hanche comprenant une tige fémorale (1) destinée à être enfouie dans le fémur à prothéser et un col prothétique (2), dont l'extrémité reçoit une tête destinée à coopérer avec un noyau de frottement destiné à être reçu dans la cavité cotyloïdienne de l'articulation considérée, *caractérisée* en ce que la tige fémorale (1) présente une section transversale de forme rectangulaire, dont le côté (3) correspondant à la corticale interne de la tige adopte un profil, dont les angles (5) s'arrondissent progressivement entre la zone distale et la zone proximale de la tige (1).

## Description

De manière connue, une prothèse de hanche est constituée d'une tige fémorale destinée à être insérée dans le canal médullaire du fémur de l'articulation de la hanche considérée, et d'un col prothétique, surmonté d'une tête, faisant ou non partie intégrante de la tige, et destiné à coopérer à son extrémité avec un noyau de frottement, le plus souvent réalisé en polyéthylène, lui-même destiné à coopérer avec la cavité cotyloïdienne de l'os iliaque de la hanche considérée, au niveau de laquelle est préalablement inséré ou mis en place un cotyle de forme appropriée.

Ces prothèses présentent cependant un certain nombre d'inconvénients s'agissant notamment des risques de descellement de la tige fémorale hors du fémur, mais également des pics de contrainte exercés au niveau notamment de la corticale interne du fémur par la tige, lesquels entraînent des douleurs chez le patient.

Plusieurs solutions ont été proposées pour résoudre ce type de difficultés. Ainsi, par exemple, le document FR-A-2 693 367 décrit une tige dont les faces principales de la partie métaphysaire présentent des décrochements successifs qui, en combinaison avec les quatre arêtes vives longitudinales résultant de la forme polygonale de la section transversale, sont sensées s'opposer aux contraintes verticales et à la rotation que peut subir la tige.

Même si les risques de descellement sont diminués, on observe cependant encore des pics de contrainte probablement dus à la présence d'arêtes vives. En outre et surtout, son architecture spécifique en fait une prothèse chère à produire.

Le document FR-A-2 686 789 décrit une tige fémorale dont la section évolue de la zone distale à la zone proximale d'une forme cylindrique à une forme elliptique, les faces de la portion proximale étant en outre dotées de rainures parallèles longitudinales de profondeur décroissante de haut en bas.

Si ce type de prothèse permet d'améliorer les problèmes de pics de contrainte du fait de la présence d'arrondis, en revanche, les difficultés liées à la rotation de la tige une fois scellée subsistent.

Le document FR-A-2 629 708 décrit une prothèse fémorale, dont la tige présente une forme évolutive de la zone distale à la zone proximale passant d'une forme circulaire à une forme dite rectangulaire dont les petits côtés sont arrondis. La tige présente en outre sur chacune des faces antérieures et postérieures correspondant au grand côté de la section rectangulaire deux nervures sensiblement parallèles partant de l'extrémité proximale jusqu'à la queue. Là encore, la présence indispensable de nervures augmente le coût de fabrication de ce type de tiges.

En d'autres termes, le problème que se propose de résoudre l'invention est de fournir une tige qui présente un risque diminué de descellement et une quasi absence de pics de contrainte.

Pour ce faire, l'invention propose une prothèse de hanche comprenant une tige fémorale destinée à être enfouie dans le fémur à prothéser et un col prothétique dont l'extrémité reçoit une tête destinée à coopérer avec un noyau de frottement destiné à être reçu dans la cavité cotyloïdienne de l'articulation considérée.

Cette prothèse se caractérise en ce que la tige fémorale présente une section transversale de forme rectangulaire, dont le côté correspondant à la corticale interne de la tige adopte un profil progressivement circulaire dans une zone comprise entre la zone distale et la zone proximale de la tige.

Plus spécifiquement, la solution technique préconisée pour répondre au problème posé consiste à munir la face interne de la tige, destinée à venir en contact avec la corticale interne du fémur, d'un profil plan, dont les arêtes latérales présentent un profil arrondi. Le rayon de courbure desdites arêtes évolue en croissant en direction de la partie métaphysaire et épiphysaire de la tige. Ce faisant, on aboutit à un blocage anti-rotation de la tige au sein du fémur, inhérent à la planéïté de la face interne de la tige, se traduisant au niveau de la partie diaphysaire par une section sensiblement rectangulaire. Ce blocage anti-rotation est néanmoins pondéré en partie métaphysaire par l'accroissement du rayon de courbure des arêtes situées de part et d'autre de la partie plane de la face interne de la tige.

Ce caractère évolutif des arêtes, et notamment de leur rayon de courbure, permet d'éviter tout risque de fracture de l'os lors de la pose de la tige. Cependant, il n'altère pas le caractère anti-rotation de la tige considérée. En outre, ce profil particulier permet de diminuer les pics de contrainte, dont il a été rappelé qu'ils sont à l'origine des douleurs chez le patient.

Ainsi, les Demandeurs ont constaté que de façon tout à fait surprenante, la section spécifique de forme évolutive de la tige permettait non seulement de lutter efficacement contre les mouvements de rotation de celle-ci, mais également de diminuer le risque d'apparition de pics de contrainte.

Dans la suite de la description et dans les revendications, la forme rectangulaire de la section recouvre également la forme carrée, laquelle constitue un rectangle particulier. En pratique, la forme de la section sera choisie en fonction de la taille de la tige, carrée pour les petites tiges et rectangulaire pour les tiges plus grandes.

Dans une forme de réalisation avantageuse, la corticale interne de la tige adopte un profil, dont les angles s'arrondissent, jusqu'à atteindre une forme quasiment circulaire de la zone métaphysaire à la zone épiphysaire.

Pour diminuer davantage encore les risques de pics de contrainte, la forme rectangulaire constitutive de la section transversale présente en outre des angles arrondis.

Selon une autre caractéristique, pour permettre une meilleure répartition des contraintes, la tige présente en outre des rayons interne et externe variables de la zone distale à la zone proximale de la tige. On entend par rayon interne le rayon de la zone de courbure de le section de la tige au niveau de la corticale interne, et par rayon externe, le rayon de la zone de courbure de la section de la tige au niveau du dos de celle-ci.

En pratique, mais de façon non limitative, les rayons interne et externe varient en fonction de la taille de la tige et sont compris respectivement entre 2,80 et 6,5 mm et entre 2 et 3,5 mm.

Pour renforcer davantage encore la répartition des contraintes, le point d'intersection de l'axe du col et de l'axe théorique vertical de la tige ne correspond pas au point d'intersection de la face externe avec le dos supérieur de la tige.

L'invention et les avantages qui en découlent ressortiront mieux de l'exemple de réalisation suivant à l'appui des figures annexées dans lesquelles :
- la figure 1 est une représentation de profil d'une tige fémorale ;
- la figure 2 est une représentation en coupe de la tige de la figure 1, selon les axes B-B, C-C, D-D, E-E, F-F et G-G de la figure 1.

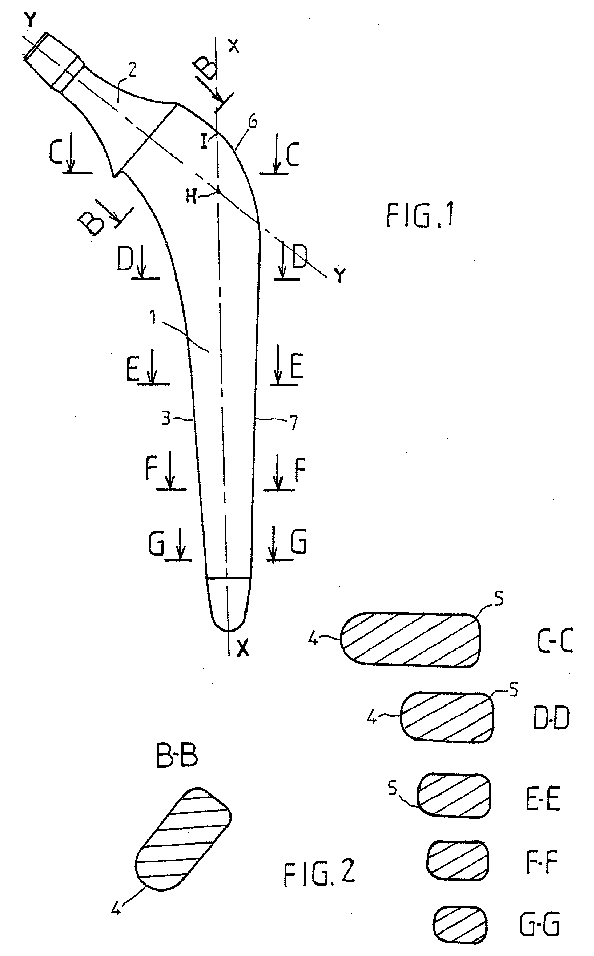

La figure 1 représente schématiquement une prothèse de hanche conforme à l'invention constituée d'une tige fémorale (1) se prolongeant à son extrémité proximale par un col prothétique (2). De manière connue, le col prothétique (2) est destiné à dépasser du fémur à prothéser et comprend dans le cas d'espèce un cône morse destiné à recevoir une tête sphérique, le plus souvent réalisée en acier inoxydable ou en céramique, et destiné à son tour à coopérer avec la cupule cotyloïdienne par l'intermédiaire d'un noyau de frottement.

Sur la figure 2, on a représenté la section transversale caractéristique de la tige de l'invention. Ainsi, la section présente une forme générale rectangulaire de la zone distale à la zone métaphysaire correspondant aux coupes E - E, F - F, G - G. Le côté (3) de la tige correspondant à zone de la tige destinée à venir au contact de la corticale interne du fémur, adopte un profil variable entre la zone métaphysaire et la zone épiphysaire, dans lequel le rayon de courbure des arêtes latérales s'arrondit, jusqu'à conférer à la tige une forme sensiblement circulaire (4) en zone épiphysaire (voir au niveau de la coupe C - C).

Selon une autre caractéristique, les angles (5) de la section transversale sont légèrement arrondis, de sorte à diminuer les risques de pics de contrainte.

En outre, la combinaison de la forme rectangulaire s'étendant de la zone distale à la zone métaphysaire, puis l'arrondi ménagé sur la corticale interne du rectangle jusqu'à obtenir une forme semi-circulaire de la zone métaphysaire à la zone épiphysaire permet d'éviter non seulement tous risques de descellement de la tige, mais également toute rotation de celle-ci.

Par ailleurs, pour obtenir comme déjà dit une meilleure répartition des contraintes, la tige présente une variation respective des rayons interne et externe de la zone distale à la zone proximale :
- pour une première série, dite série 1, entre 2,80 et 3,80 mm, et entre 2 et 3 mm ;
- pour une deuxième série, dite série 2, entre 3 et 5,5 mm, et entre 2,5 et 3 mm ;
- pour une troisième série, dite série 3, entre 3 et 6,5 mm, et entre 2,5 et 3 mm ;
- et pour une quatrième série, dite série 4, entre 3,5 et 6,10 mm et entre 3, et 3,50 mm.

Ces différentes séries sont destinées à permettre au chirurgien de sélectionner la prothèse la mieux adaptée en fonction de critères anatomiques et biomécaniques. Par ailleurs, la déclinaison au sein de chaque série, de une à plusieurs prothèses, permet de faire varier la taille de remplissage du fémur à prothéser.

On a représenté à titre d'exemple dans le tableau ci-dessous, l'évolution des rayons interne et externe de la tige au niveau de chacune des coupes pour une prothèse de la série 4C.

| **Axe** | **Rayon Interne (mm)** | **Rayon externe (mm)** |
|---|---|---|
| B - B | 6,05 | 3,47 |
| C - C | 6,10 | 3,5 |
| D - D | 5,24 | 3,3 |
| E - E | 4,42 | 3,21 |
| F - F | 3,17 | 3,10 |
| G-<G | 3,57 | 3,01 |

Selon une autre caractéristique de l'invention, apparaît sur la figure 1 le point H correspondant au point d'intersection de l'axe vertical théorique X-X et l'axe du col Y-Y, de même que le point I correspondant au point d'intersection du dos supérieur (6) avec la face externe (7). Comme déjà dit, la non superposition des points H et I conduit à améliorer la répartition des contraintes.

Les avantages de l'invention ressortent bien de la description qui précède. On notera plus particulièrement l'absence de risque de descellement et la diminution des pics de contrainte.

## Revendications

1. Prothèse de hanche comprenant une tige fémorale destinée à être enfouie dans le fémur à prothéser et un col prothétique, dont l'extrémité reçoit une tête destinée à coopérer avec un noyau de frottement destiné à être reçu dans la cavité cotyloïdienne de l'articulation considérée, ***caractérisée* en ce que** la tige fémorale présente une section transversale de forme rectangulaire, dont le côté (3) destiné à venir en contact avec la corticale interne du fémur de l'articulation considérée adopte un profil plan entre la zone diaphysaire et la zone métaphysaire, dont le rayon de courbure des arêtes latérales s'accroît entre la zone distale et la zone proximale de la tige.

2. Prothèse selon la revendication 1, ***caractérisée* en ce que** le côté du rectangle destiné à venir en contact avec la corticale interne du fémur de l'articulation considérée adopte un profil atteignant progressivement une forme sensiblement circulaire au niveau de la zone épiphysaire de la tige.

3. Prothèse selon la revendication 1, ***caractérisée* en ce que** les angles du rectangle constitutifs de la section transversale sont arrondis.

4. Prothèse selon la revendication 1, ***caractérisée* en ce qu'**elle présente des rayons interne et externe, variables de la zone distale à la zone proximale.
